# EUROPEAN PATENT APPLICATION

(11) **EP 3 527 207 A1**
(43) Date of publication of application: **21.08.2019**
(21) Application number: 17862701.4
(22) Date of filing: 17.10.2017
(51) Int. Cl.: A61K 31/231, A23L 33/10

(54) **COMPOSITION FOR PREVENTING OR TREATING PSORIASIS CONTAINING MONOACETYL DIACYLGLYCEROL COMPOUND**

(30) Priority: 17.10.2016 KR 20160134296
(71) Applicant: Enzychem Lifesciences Corporation, Daejon 34051 (KR)
(72) Inventor: SOHN, Ki Young, Seoul 07264 (KR); YOON, Sun Young, Daejeon 34199 (KR); LEE, Har Eum, Daejeon 34207 (KR)
(74) Representative: Algemeen Octrooi- en Merkenbureau B.V.
(86) International application number: PCT/KR2017/011449
(87) International publication number: WO 2018/074810

(57) **Abstract**

Disclosed is a composition for preventing or treating psoriasis containing a monoacetyl diacylglycerol compound which not only can effectively prevent and treat psoriasis, but also is safe without any side effects when used. The composition contains a monoacetyl diacylglycerol compound represented by Chemical Formula 1 in the specification as an active ingredient.

## Description

### [TECHNICAL FIELD]

The present invention relates to a composition for preventing or treating psoriasis containing a monoacetyl diacylglycerol compound. More specifically, the present invention relates to a composition for preventing or treating psoriasis containing a monoacetyl diacylglycerol compound which not only can effectively prevent and treat psoriasis, but also is safe without any side effects when used.

### [BACKGROUND ART]

Over the past few years, many articles have revealed the role of Th17 cells (T helper 17 cells) and their specific effector cytokine known as IL-17(Interleukin 17) in various autoimmune diseases such as rheumatoid arthritis, psoriasis, psoriatic arthritis (PsA), systemic lupus erythematous (SLE) and multiple sclerosis, etc. It has been known that IL-17 is associated with skin inflammation in psoriasis.

Among them, psoriasis is a common skin inflammatory disease, estimated to account for about 2% of the world's population. Psoriasis is a chronic inflammatory skin disease characterized by an increase in hyperproliferative keratinocytes and scales on the skin, and is an immune-mediated autoimmune skin disease induced by chronic activation of inflammatory cell infiltration in the skin and dysregulation of epidermal keratinocytes. Psoriasis typically appears on the areas that often receive irritation, such as elbows, knees, hips, scalp, etc., which are characterized by erythematous skin lesions covered with a silvery-white scale with a clear border, and is a chronic inflammatory skin disease with various clinical features ranging from small papules to plaque psoriasis, inverse psoriasis, guttate psoriasis, pustular psoriasis, erythrodermic psoriasis, exfoliative psoriasis, and psoriatic arthritis. Histologically, it is characterized by epidermal thickening, scaling, and hyperproliferative parakeratosis in skin biopsy.

The pathogenesis of psoriasis will be briefly described as follows: First, immune cells are activated by pathogens, and activated immune cells express STAT3, IL-23, etc., and keratinocytes are activated by these factors. Activated keratinocytes express chemokines such as CXCL1 and CXCL2, which induce activation and recruitment of neutrophils, ultimately leading to psoriasis. IL-23 mainly activates Th17 cells and also plays an important role in inducing differentiation and maintaining Th17 cells. Further, STAT3 also activates Th17 cells.

STAT is a transcription factor related to autoimmune diseases, which plays an important role in cell-mediated immune responses induced by cytokines, and is crucial to the cytokine receptor signaling system. STAT3 is induced by stimulation of IL-6 and IL-10 (Frucht DM, et al., J Immunol 2000: 4659-64). It has been known that STAT3 is involved in cell growth and proliferation, and when activated, it translocates to the cell nucleus to induce expression of proteins involved in growth and proliferation of various cells. Further, it activates Th17 cells known as a new lineage of CD4+T cells. STAT3 acts directly on the differentiation and signaling of Th17 cells, thereby modulating RORrt, which is a specific transcription factor for Th17 cells (Onai N, *et al.,* Immunity 2008: 490-492).

TGFβ has been known to function in suppressing proliferation of keratinocytes in psoriasis. Recently, it has been reported that overexpression of TGFβ1 is associated with psoriasis-like skin inflammation, and also, it is estimated that TGFβ1 is involved in the angiogenesis, chemotaxis of inflammatory cells, and differentiation of Th17 cell, and thereby plays an important role in the pathogenesis of psoriasis.

Th17 cells, typical proinflammatory cells, secrete IL-17, IL-17F, IL-21 and IL-22. Th17 cells infiltrated into peripheral tissues act on macrophages, dendritic cells, fibroblasts, vascular endothelial cells, osteoclasts, etc., and thereby cause tissue damage by secreting various inflammatory cytokines (IL-1, TNF, IL-8, IL-6, etc.) and chemokines. In particular, keratinocytes are activated through secretion of IL-17 and IL-22, and activated keratinocytes produce many inflammatory cytokine/chemokine precursors. Th17 cells have been found to be the main pathological cells mediating inflammatory diseases, and it has been reported that Th17 cells and Th-17-mediated cytokines such as IL-17A, IL-22 and IL-23 trigger psoriasis. Th17 cells can rapidly initiate inflammatory responses dominated by neutrophils (Miossec, et al. 2009, NEJM; 361:888-98) .

IL-17 is secreted from Th17 cells and consists of IL-17A, IL-17B, IL-17C, IL-17D, IL-17E(IL-25) and IL-17F, and these proteins all have similar protein structures. IL-17 is a type of inflammatory cytokine and activates NF-_{K}B and MAP kinases of immune cells and promotes secretion of IL-6, PTGS2/COX-2 and nitric oxide (Rouvier, et al., J. Immunol., vol. 150, no. 12, 1993, pages 5445-56). Similar to IFN-γ, IL-17 is a cytokine that acts as a potent mediator in a delayed-type response by increasing chemokine production in various tissues so as to recruit monocytes and neutrophils into inflammatory sites. The IL-17 family functions as an inflammation-inducing cytokine that responds to the invasion of the immune system by extracellular pathogens and induces destruction of cellular matrix of pathogens (Miossec P, et al. 2009, N. Engl. J. Med. 361:888-98). The chemotaxis by chemokines whose production is increased by IL-17 induces neutrophil recruitment and ultimately induces excessive inflammatory skin diseases.

Common treatments for psoriasis include topical medication, phototherapy and internal medication. In topical treatments, steroids, coal tar, anthralin, vitamin D3 and analogs thereof, retinoids, sunlight, etc., are used. These topical treatments have side effects of skin thinning, chapped skin, burns, irritation, and photosensitivity. In addition, steroids may induce tolerance and thus affect subsequent steroid therapies. Phototherapy involves the administration of psoralen in combination with ultraviolet B or A, but has disadvantages in that skin aging may be promoted and the incidence of skin cancer may be increased. In addition, the internal medication applied to the most severe psoriasis involves the administration of immunomodulators, such as cyclosporine. However, nephrotoxicity or hypertension may occur upon long-term use of cyclosporine, and thus, careful observation is needed. The existing treatments for psoriasis have several side effects.

Therefore, although psoriasis typically progresses to moderate to severe levels, there have not yet been developed a specific treatment method which can be efficiently used in the prevention and treatment of psoriasis, which is effective and does not cause side effects.

### [DETAILED DESCRIPTION OF THE INVENTION]

### [Technical Problem]

Accordingly, it is one object of the present invention to provide a composition for preventing or treating psoriasis containing a monoacetyl diacylglycerol compound which can effectively prevent or treat psoriasis without side effects.

### [Technical Solution]

In order to achieve the object above, one aspect of the present disclosure provides a pharmaceutical composition for preventing or treating psoriasis containing a monoacetyl diacylglycerol compound represented by the following Chemical Formula 1 as an active ingredient: 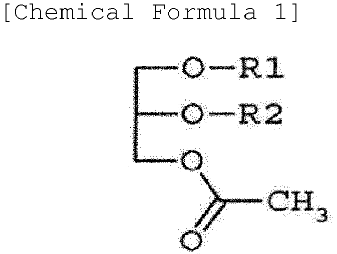 in Chemical Formula 1, R₁ and R₂ are each independently a fatty acid group having 14 to 22 carbon atoms.

Another aspect of the present disclosure provides a health functional food composition which includes the aforementioned composition and is capable of preventing or improving psoriasis.

Still another aspect of the present disclosure provides a method for preventing or treating psoriasis including administering the aforementioned composition to a subject suspected of psoriasis.

### [ADVANTAGEOUS EFFECTS]

The composition according to the present invention is not only effective in preventing and treating psoriasis, but also can be safely used without side effects.

### [BRIEF DESCRIPTION OF THE DRAWINGS]

FIG. 1 is a diagram showing an example of a mechanism for preventing psoriasis using PLAG of the present invention.
FIG. 2 is an overall schematic diagram of the experiment of the present invention.
FIG. 3 shows an image (A) and a graph (B) illustrating the effect of EC-18 in keratinization. Vaseline is the normal control group (hereinafter, referred to as control), IMQ is the psoriasis-induced group (hereinafter, referred to as psoriasis-induced group), and IMQ + EC-18 is the sample-administered group (hereinafter, referred to as PLAG group).
FIG. 4 shows an image (A) and a graph (B) illustrating the effect of EC-18 on the size and weight of spleen.
FIG. 5 shows images illustrating the effect of EC-18 on the epidermal thickness of the back skin.
FIG. 6 shows images illustrating the effect of EC-18 on neutrophil recruitment in the back skin.
FIG. 7 shows images illustrating the effect of EC-18 on IL-17 expression in the back skin.
FIG. 8 shows graphs illustrating the effect of EC-18 on the expression of IL-6 and IL-17 in serum.

### [DETAILED DESCRIPTION OF THE EMBODIMENTS]

Hereinafter, the present invention will be described in more detail with reference to the accompanying drawings.

The pharmaceutical composition for preventing or treating psoriasis contains a monoacetyl diacylglycerol compound represented by the following Chemical Formula 1 as an active ingredient: 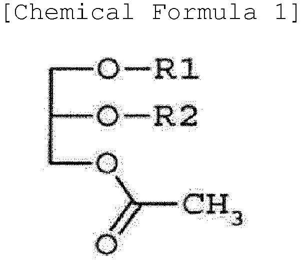 in Chemical Formula 1, R₁ and R₂ are each independently a fatty acid group having 14 to 22 carbon atoms, preferably a fatty acid group having 15 to 20 carbon atoms.

As used herein, the term "monoacetyl diacylglycerol compound" refers to a glycerol derivative having one acetyl group and two acyl groups, and is also simply referred to as monoacetyl diacylglycerol (MADG). In Chemical Formula 1, R₁ and R₂ are each independently a fatty acid group having 14 to 22 carbon atoms (in fatty acids, it refers to the remaining part from which the hydroxy group is excluded), and may be preferably palmitoyl, oleoyl, linoleoyl, linolenoyl, stearoyl, myristoyl and arachidonoyl, but is not limited thereto. More preferably, the combination of R₁ and R₂ may include oleoyl/palmitoyl, palmitoyl/oleoyl, palmitoyl/linoleoyl, palmitoyl/linolenoyl, palmitoyl/arachidonoyl, palmitoyl/stearoyl, palmitoyl/palmitoyl, oleoyl/stearoyl, linoleoyl/palmitoyl, linoleoyl/stearoyl, stearoyl/linoleoyl, stearoyl/oleoyl, myristoyl/linoleoyl, or myristoyl/oleoyl, and most preferably, the combination of R₁ and R₂ may be palmitoyl/ linoleoyl. Further, in terms of optical activity, the monoacetyl diacylglycerol compound may be (R)-form, (S)-form or a racemic mixture, and may be preferably a racemic mixture. Also, all stereoisomers of the above-mentioned compound are within the scope of the present invention.

For example, the monoacetyl diacylglycerol compound may be a compound represented by the following Chemical Formula 2:

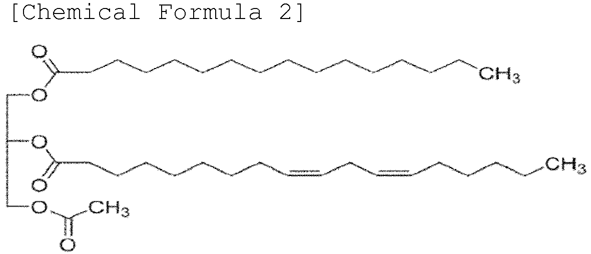

The compound represented by Chemical Formula 2 is 1-palmitoyl-2-linoleoyl-3-acetyl-rac-glycerol, which corresponds to the compound where R₁ and R₂ of Chemical Formula 1 are palmitoyl and linoleoyl, respectively. It is also named as "PLAG" or "EC-18" as needed.

The monoacetyl diacylglycerol compound of the present invention may be extracted/separated from deer antlers or may be produced by known organic synthesis methods. For example, a deer antler is extracted with hexane, and extracting the extraction residue with chloroform, followed by subjecting the thus-obtained extraction liquid to distillation under reduced pressure to obtain chloroform extracts. The hexane and chloroform as the solvents used for the extraction are used in an amount sufficient to immerse the deer antler. In general, about 4 to 5 liters of each of hexane and chloroform is used for 1 kg of deer antler, but the type of extraction solvents and amount thereof are not limited thereto. The chloroform extracts of the deer antler obtained by this method is further fractionated and purified by silica gel column chromatography or TLC to obtain the monoacetyl diacylglycerol compound used in the present invention. As an eluent used in the chromatography purification step, chloroform/methanol, hexane/ethyl acetate, and hexane/ethyl acetate/acetic acid may be used, but is not limited thereto.

One example of the method for chemically synthesizing the monoacetyl diacylglycerol compound is disclosed in Korean Patent No. 10-0789323. According to the method of this patent, the desired monoacetyl diacylglycerol compound can be synthesized by the steps of: (a) preparing 1-R₁-3-protecting group-glycerol by adding a protecting group at the position 3 of 1-R₁-glycerol; (b) preparing 1-R₁-2-R₂-3-protecting group-glycerol by introducing R₂ at the position 2 of 1-R₁-3-protecting group-glycerol; and (c) performing a deprotection reaction and acetylation reaction of 1-R₁-2-R₂-3-protecting group-glycerol at the same time. Alternatively, acetolysis of phosphatidylcholine may be used, but is not limited thereto.

The monoacetyl diacylglycerol compound exhibits excellent activities in improving, preventing or treating psoriasis. It was confirmed that the monoacetyl diacylglycerol compound can reduce the secretion of cytokines selected from the group consisting of IL-6, IL-10, IL-17, and IL-22, and thus can be effectively used in the prevention or treatment of psoriasis.

The content of the monoacetyl diacylglycerol compound contained in the pharmaceutical composition of the present invention is not particularly limited, but is preferably contained in an amount of 0.0001 to 100.0% by weight, preferably 0.001 to 50% by weight, more preferably 0.01 to 20% by weight, based on the total weight of the composition. If desired, the pharmaceutical composition of the present invention may further include other active ingredients having a therapeutic effect of psoriasis. The pharmaceutical composition may have a formulation such as tablets, pills, powders, granules, capsules, suspensions, solutions, emulsions, syrups, sterile aqueous solutions, non-aqueous solvents, suspensions, freeze-dried preparations, suppositories, etc., and may be formulated for oral or parenteral administration. The composition of the present invention may be formulated with commonly used diluents or excipients, such as fillers, extenders, binders, wetting agents, disintegrants, surfactants, etc. Therefore, the pharmaceutical composition of the present invention may further include carriers, excipients, diluents, etc., which are conventionally used in the production of pharmaceutical compositions. Solid formulations for oral administration include tablets, pills, powders, granules, capsules, etc., and these solid formulations are prepared by mixing one or more active compounds with at least one excipient, for example, starch, calcium carbonate, sucrose, lactose, gelatin, etc. In addition to simple excipients, lubricants such as magnesium stearate, talc, etc. may also be used. Liquid formulations for oral administration include suspensions, solutions, emulsions, syrups, etc., and may contain various excipients, for example, wetting agents, flavoring agents, aromatics and preservatives, in addition to water and liquid paraffin, which are frequently used-simple diluents. Formulations for parenteral administration include sterilized aqueous solutions, non-aqueous solutions, suspensions, emulsions, freeze-dried preparations, suppositories, *etc*. As non-aqueous solvents or suspending agents, propylene glycol, polyethylene glycol, vegetable oils such as olive oil, injectable esters such as ethyl oleate, etc. can be used. As the base of the suppositories, witepsol, Macrogol, Tween 61, cacao butter, laurin fat, glycerogelatin, etc. can be used.

The compound of the present invention may be administered in a pharmaceutically effective amount. As used herein, the term "pharmaceutically effective amount" refers to an amount sufficient to treat diseases, at a reasonable benefit/risk ratio applicable to any medical treatment. The effective dosage level of the composition may be determined depending on the subject's type, age, sex, weight, the type of a disease, the severity of a disease, the activity of a drug, the sensitivity to a drug, the form of a drug, the route of administration, the duration of administration, the excretion rate, drugs used in combination with the composition, and other factors known in the medical field. The composition of the present invention may be administered as an individual therapeutic agent or in combination with other therapeutic agents, and may be administered sequentially or simultaneously with other therapeutic agents. Also, the composition may be administered in a single or multiple dosage form. It is important to administer the composition in the minimum amount that can exhibit the maximum effect without causing side effects, in consideration of all the above-described factors, and this amount can be easily determined by a person skilled in the art. The preferred dose of the composition of the present invention may be determined by one who performs treatments within the scope of appropriate medical decisions, and may be typically administered at a dose of 0.01 to 4000 mg/kg, preferably 0.05 to 2000 mg/kg, and more preferably 0.1 to 1000 mg/kg once or in a few divided doses in a day. For example, the composition may be administered once or several times at a dose of 1 mg to 4 g, preferably 30 mg to 2 g per day for an adult with a body weight of 60 kg. Since the composition of the present invention has no toxicity and side effects, it can be administered for a long period of time for the purpose of prevention or treatment. The composition may be applied to any subject as long as the purpose is to prevent or treat psoriasis, without particular limitations. Examples of the subject include human, non-human animals such as monkeys, dogs, cats, rabbits, marmots, rats, mice, cows, sheep, pigs, and goats, etc., and mammals. The route of administration includes any method as long as it is a conventional method in the art. For example, the composition may be administered via oral route, but is not limited thereto.

Further, the present invention provides a health functional food composition which contains a monoacetyl diacylglycerol compound represented by the following Chemical Formula 1, as an active ingredient, and is capable of preventing or improving psoriasis: 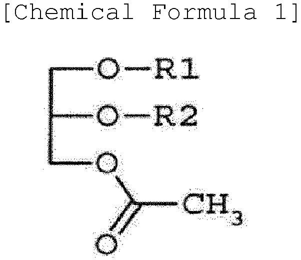 in Chemical Formula 1, R₁ and R₂ are each independently a fatty acid group having 14 to 22 carbon atoms.

That is, by incorporating the monoacetyl diacylglycerol compound of the present invention into the health functional food composition, psoriasis of the subject may be prevented or improved. The monoacetyl diacylglycerol compound and psoriasis are as described above. When the compound of the present invention is incorporated into the health functional food composition, the mixed amount of the active ingredients may be appropriately determined depending on the intended use. The compound of the present invention itself may be used as a health functional food composition. In this case, the composition and content of the composition may be the same as the composition and content of the pharmaceutical composition. At this time, the content of the compound of the present invention may be, for example, 0.0001 to 100.0% by weight, preferably 0.001 to 50% by weight, more preferably 0.01 to 20% by weight, based on the total weight of the composition. Further, in general, when a health functional food is prepared by mixing the compound of the present invention with a food or beverage, the compound of the present invention is not particularly limited by the type of raw materials (food or beverage) of the health functional food, and specifically, the health functional food may include meats, sausages, bread, chocolates, candies, snacks, confectionery, pizza, instant noodles, other noodles, gums, daily products including ice creams, various soups, beverages, teas, drinks, alcoholic beverages, and vitamin complexes, and may also include any conventional health functional food and animal feeds.

In addition, when the health functional food composition is used in the form of a beverage, it may contain conventional sweeteners, flavoring agents, natural carbohydrates, etc., as additional ingredients. Examples of the natural carbohydrates include monosaccharides such as glucose and fructose, disaccharides such as maltose and sucrose, polysaccharides such as dextrin and cyclodextrin, and sugar alcohols such as xylitol, sorbitol and erythritol. The ratio of the natural carbohydrates may preferably be about 0.01 to 0.04 g, more preferably 0.02 to 0.03 g per 100 mL of the composition of the present invention. Examples of the sweeteners include natural sweeteners such as thaumatin and stevia extracts, and artificial sweeteners such as saccharin and aspartame. In addition to the above, the health functional food composition of the present invention may contain various nutrients, vitamins, electrolytes, flavoring agents, colorants, pectic acid and salts thereof, alginic acid and salts thereof, organic acids, protective colloidal thickening agents, pH controlling agents, stabilizing agents, preservatives, glycerin, alcohol, carbonizing agents used in carbonated beverages, etc. Moreover, the health functional food composition of the present invention may contain fruits as used in preparing natural fruit juices, fruit juice beverages and vegetable beverages.

In addition, the present invention provides a method for preventing or treating psoriasis including:
administering the pharmaceutical composition to a subject suspected of psoriasis. That is, by administering the compound of the present invention to subject suspected of psoriasis, psoriasis can be efficiently prevented or treated. As used herein, the subject suspected of psoriasis refers to a subject who has psoriasis or has the potential of having psoriasis. In the therapeutic method of the present invention, the type of monoacetyl diacylglycerol compound, the dose of monoacetyl diacylglycerol compound, and psoriasis are as described above. As used herein, the term "administration" refers to introducing the pharmaceutical composition of the present invention to a subject suspected of psoriasis via any appropriate method. The administration route may include various oral or parenteral routes as long as the composition can reach a desired tissue, and for example, oral administration, transdermal administration (topical application, etc.), intravenous administration, intramuscular administration, subcutaneous administration, intradermal administration, etc. may be used, but is not limited thereto. As used herein, the term "prevention" refers to any activity that inhibits or delays occurrence of psoriasis by administering the composition of the present invention. As used herein, the term "treatment" refers to any activity that alleviates or positively changes symptoms of psoriasis by the composition of the present invention. As used herein, the term "improvement" refers to any activity that alleviates or ameliorates symptoms of subjects suspected of or having psoriasis using the composition of the present invention.

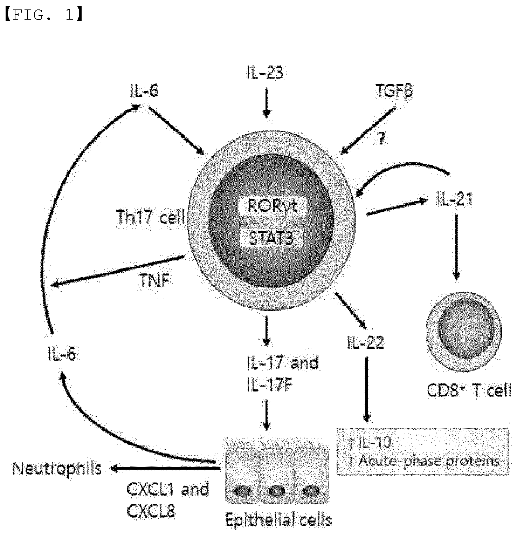

FIG. 1 is a diagram showing an example of a mechanism for preventing psoriasis using PLAG of the present invention. As shown in FIG. 1, Th17 cells are stimulated by IL-6, IL-23, IL-21 and TGF-β, and the stimulated Th17 cells express IL-17, IL-17F, IL-21, IL-22 and TNF. The expressed IL-17 and IL-17F stimulate epithelial cells to induce the expression of IL-6 and chemokines such as CXCL1 and CXCL8, and the expressed IL-6 again stimulates Th17 cells, mediating a positive feedback, and neutrophils are recruited by the chemokines expressed from the activated epithelial cells. When the composition of the present invention is administered by the above mechanism, the activities of IL-6 and IL-17 are inhibited and the migration of neutrophils from the blood to the skin is blocked, thereby preventing psoriasis.

Hereinafter, the present invention will be described in more detail by way of Examples. However, the present invention is not limited to or by the Examples.

In order to confirm the preventive and therapeutic effects of psoriasis by 1-palmitoyl-2-linoleoyl-3-acetylglycerol (EC-18 or PLAG), imiquimod (hereinafter, referred to as IMQ), which is commonly used to induce skin inflammation i.e., psoriasis, such as infiltration of immune cells, epidermal hyperplasia, etc., was used as a ligand for TLR7 and TLR8 to carry out an experiment using psoriasis-induced mouse models. As an experiment model, 6 to 8-week-old female Balb/c-based mice (KOATECH, Pyeongtaek, Korea) were maintained under a specific pathogen free (SPF) environment and used. PLAG (ENZYCHEM LIFESCIENCES, Korea) was prepared before *in vivo* or *in vitro* treatment. Also, statistical analysis was performed using a paired t-test. IMQ-treated mice exhibited psoriasis-like inflammation such as keratinization, increase in epidermal thickness and formation of scales.

### [Example 1] Preparation of Control Group, Psoriasis-Induced Group and PLAG group

Mice were randomly divided into three groups: control group, psoriasis-induced group, and PLAG group. As the IMQ, Aldara cream (3M Health Care Limited, UK) containing 5% of IMQ was used.

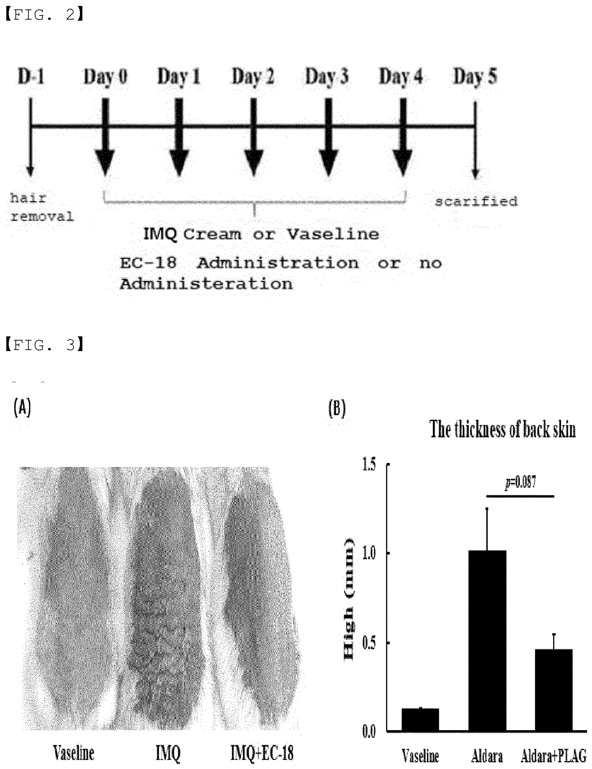

As shown in FIG. 2, the day before the experiment (D-1), and the hair on the back of the mice was removed using a hair removal cream. For 5 days from day 0 to day 4, the control group was treated with vaseline (Unilever, UK) and PBS, the psoriasis-induced group was treated with Aldara cream and PBS, and the PLAG group was treated with Aldara cream and PLAG. Vaseline and Aldara cream were applied to the back of mice at a dose of 40 mg/day, and PBS and PLAG were orally administered at a dose of 250 mg/kg/day. On Day 5, which was the 6^{th} day, the mice were sacrificed.

### [Example 2] Measurement of Keratinization and Thickness of Back Skin of Mice

In order to investigate whether PLAG prevents the progression of psoriasis, psoriasis was induced using IMQ cream (Aldara cream) as in Example 1. As shown in FIG. 3(A), the back of the mice treated as in Example 1 was photographed on day 5. Subsequently, for the measurement of thickness of back skin, the back skin was removed, and the thickness of the removed back skin was measured using a caliper. The thickness of the measured back skin is shown in the graph of FIG. 3(B).

As shown in FIG. 3(A), psoriasis was induced on the back of the mice in the psoriasis-induced group, which was confirmed by the occurrence of severe keratosis. Meanwhile, in the PLAG group, keratosis was remarkably alleviated when compared to the psoriasis-induced group. In addition, as shown in the graph of FIG. 3(B), it was confirmed that the thickness of the back skin of the psoriasis-induced group was remarkably thicker than that of the control group, while the thickness of the back skin of the PLAG group was significantly decreased. Thus, it can be seen that PLAG inhibits the progression of psoriasis and has a therapeutic effect on inflammatory skin diseases.

### [Example 3] Measurement of Size and Weight of Spleen

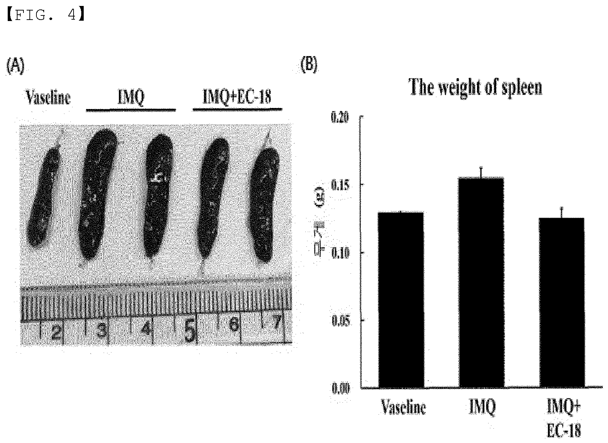

The size and weight of the spleen tend to increase when inflammation is induced using a positive control substance (*i.e.,* IMQ) that induces psoriasis, and also, the size and weight of the spleen are generally used as indicators of toxicity or the degree of an inflammatory response in a drug experiment. Thus, an increase in size and weight of the spleen in the mice may indicate an increase in inflammation in the mice. Accordingly, the spleen of the mice of Example 1 was removed, and the size and weight of the spleen were compared. The results are shown in FIG. 4.

The size of the spleen removed from the mice of Example 1 was measured using a ruler, and the weight was measured using a scale. The measurement results are shown in FIGS. 4(A) and 4(B). As shown in the image of FIG. 4(A), the size of spleen of the two groups treated with IMQ was increased compared to the control group. However, as shown in the graph of FIG. 4(B), the weight of spleen of the PLAG group was maintained the same as the weight of spleen of the control group. From these results, it was confirmed that PLAG can exhibit an effect of inhibiting inflammatory responses.

### [Example 4] Inhibition of IMQ-induced Chronic Inflammatory Skin Disorder of PLAG

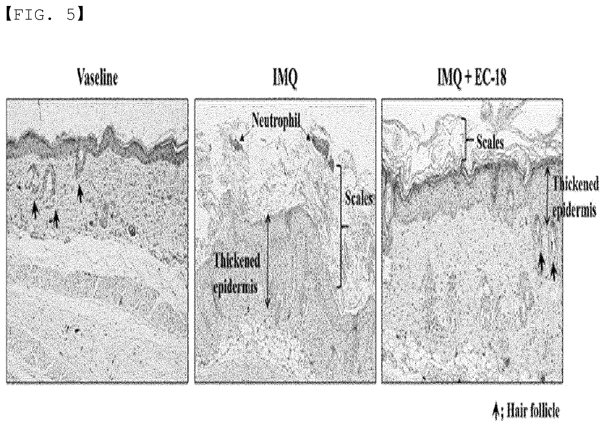

The thickness of the back skin of the mice of Example 1 was stained with the following H&E (Hematoxylin and Eosin) staining method, and histologically observed and evaluated under an optical microscope (Olympus, Tokyo, Japan) 5. The images were photographed at an X100 magnification and are shown in FIG. 5. The H&E staining method is as follows:
The back skin tissue of the mice was fixed in a 10% buffered formalin solution for 24 hours, embedded in paraffin and cut into 4µm-thick sections. After removing paraffin and rehydrating the sections, the sections were stained with hematoxylin and eosin.

FIG. 5 shows images illustrating the degree of increase in epidermal thickness by the formation of scales and proliferation of keratinocytes in the back skin of each group of mice in Example 1 above. As shown in FIG. 5, the back skin of the mice in the control group had a structure in which thickness of epidermis was thin and the hair follicles were uniformly positioned, and the skin keratinocytes (scales) were also thin. Unlike the control group, in the psoriasis-induced group, the epidermal thickness became thicker, and epidermal hyperplasia occurred in the epidermis. Further, the number of hair follicles were also reduced, the number of skin keratinocytes (scales) was increased, the stratum corneum was thickened, and inflammatory cells, i.e., neutrophils, were found between the stratum corneum. In contrast, in the PLAG group, when compared with the psoriasis-induced group, the thicknesses of the stratum corneum and the epidermis were remarkably reduced, hair follicles were present as in the control group, and neutrophils were not found. Thus, it can be seen that administration of PLAG inhibits psoriasis caused by IMQ and regulates inflammation.

### [Example 5] Histological analysis of recruitment of inflammatory cells

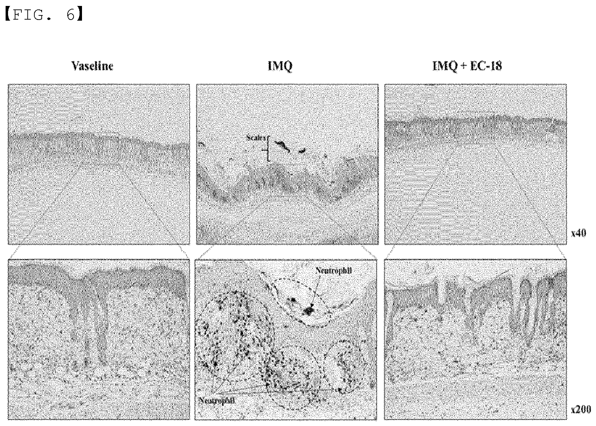

The degree of neutrophil recruitment in the back skin of the mice of Example 1 is shown in FIG. 6 through immunohistochemical staining.

Neutrophil staining by immunohistochemical staining was performed as follows: The sections prepared in Example 4 above were incubated with primary rat anti-mouse neutrophil antibodies (NIMP-R14, Thermo Fisher Scientific Inc.) (1:1000) overnight at 4 °C. After washing with TBS (Tris buffered saline), the slides were incubated for 15 minutes at room temperature with horseradish peroxidase-conjugated goat-anti-mouse IgG secondary antibodies (Santa Cruz Bioechnology) diluted at a ratio of 1:250. The tissue sections were immersed in 3-amino-9-ethylcarbazole (AEC, Dako, Denmark) as a substrate and samples were counterstained with 10% Mayer's hematoxylin, dehydrated and fixed with Crystal Mount. The irrelevant goat IgG having the same isotype and antibody diluent were served as a negative control. The cells were observed under an optical microscope (Olympus, Tokyo, Japan) and photographed at X40 and X200 magnifications, and the results are shown in FIG. 6.

As shown in FIG. 6, when compared to the control group, the back skin of the mice in the psoriasis-induced group showed an increase in neutrophil recruitment, and the level of neutrophil recruitment in the PLAG group was significantly lower than that in the psoriasis-induced group. From these results, it can be seen that PLAG inhibits the inflammatory responses of psoriasis through the inhibition of neutrophil recruitment in the skin, thereby playing a role in protecting the skin.

### [Example 6] Inhibition of IL-17 Expression in IMQ-induced Psoriatic Skin of PLAG

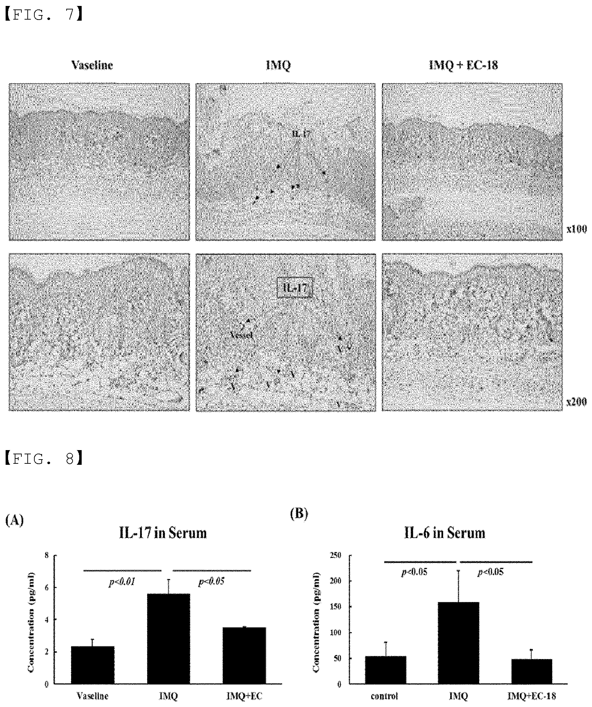

The expression level of IL-17 in the back skin of the mice of Example 1 was confirmed through immunohistochemical staining. The immunohistochemical staining was carried out in the same manner as in Example 5 except that anti-mouse IL-17 antibody (eBioscience) was used as the primary antibody instead of NIMP-R14 antibody. The cells were observed under an optical microscope (Olympus, Tokyo, Japan) and photographed at X100 and X200 magnifications, and the results are shown in FIG. 7.

As shown in FIG. 7, in the IMQ-treated back skin, the IL-17 expression was locally increased in the blood vessels by the immunohistochemical staining. Whereas in the mice of the PLAG group, the IL-17 expression was significantly inhibited in the skin blood vessels, showing the expression level similar to the control group. Thus, it was confirmed that EC-18 plays a role in regulating the expression of IL-17, which is associated with skin inflammation in psoriasis, and thereby inhibits psoriasis induced by IMQ.

### [Example 7] Inhibition of IL-6 and IL-17 in Serum of IMAG-Induced Psoriatic Mice of PLAG

The level of IL-17 and IL-6 expression in the serum of the mice of Example 1 was confirmed. All the experiment results were statistically analyzed using Student's t-test. Significance was verified at p <0.01 and p <0.05 or less, when statistically significant. The serum was collected from the mice in the control group, the drug-administered psoriasis-induced group, and the group in which PLAG (EC-18) was administered along with the drug-administered group, and the concentrations of IL-17 and IL-6 present in the serum were measured by ELISA assay.

FIG. 8 shows graphs illustrating the effects of reducing the expression and secretion of IL-17 and IL-6 proteins, which were increased in the serum of IMQ-induced psoriatic mice, when the composition of the present invention was administered. As shown in FIG. 8, the expression level of IL-17 and IL-6 present in the serum was increased by 2 to 3-fold in the mouse group in which psoriasis was induced by IMQ, compared to the control group. The group administered together with PLAG showed an expression level similar to that of the control group and no change was observed.

## Claims

1. A pharmaceutical composition for preventing or treating psoriasis comprising a monoacetyl diacylglycerol compound represented by the following Chemical Formula 1 as an active ingredient: 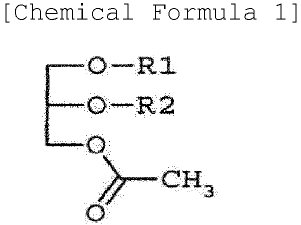 in Chemical Formula 1, R₁ and R₂ are each independently a fatty acid group having 14 to 22 carbon atoms.

2. The pharmaceutical composition for preventing or treating psoriasis of claim 1, wherein the R₁ and R₂ are each independently selected from the group consisting of palmitoyl, oleoyl, linoleoyl, linolenoyl, stearoyl, myristoyl and arachidonoyl.

3. The pharmaceutical composition for preventing or treating psoriasis of claim 1, wherein the monoacetyl diacylglycerol compound is represented by the following Chemical Formula 2.

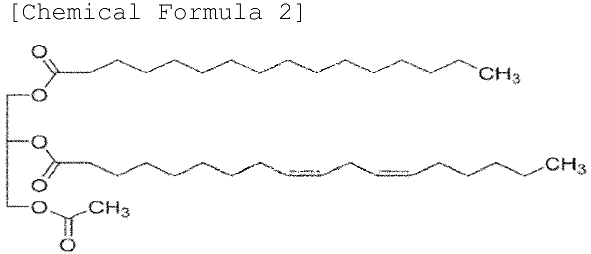

4. The pharmaceutical composition for preventing or treating psoriasis of claim 1, wherein the monoacetyl diacylglycerol compound is isolated from a deer antler.

5. The pharmaceutical composition for preventing or treating psoriasis of claim 1, wherein the monoacetyl diacylglycerol compound inhibits the expression and/or activity of any one or more cytokines selected from the group consisting of IL-6, IL-10, IL-17 and IL-22.

6. The pharmaceutical composition for preventing or treating psoriasis of claim 5, wherein the monoacetyl diacylglycerol compound inhibits the expression and/or activity of IL-17 among the cytokines.

7. The pharmaceutical composition for preventing or treating psoriasis of claim 6, wherein the IL-17 induces the expression of IL-6 and chemokines of epithelial cells and/or recruitment of neutrophils.

8. A health functional food composition comprising a monoacetyl diacylglycerol compound represented by the following Chemical Formula 1, as an active ingredient, and is capable of preventing or improving psoriasis: 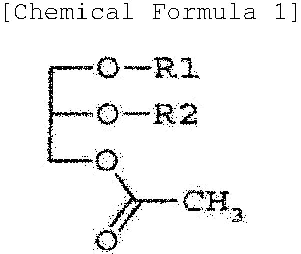 in Chemical Formula 1, R₁ and R₂ are each independently a fatty acid group having 14 to 22 carbon atoms.

9. A method for preventing or treating psoriasis comprising:
administering the composition of any one of claims 1 to 8 to a subject suspected of psoriasis.

10. The method for preventing or treating psoriasis of claim 9, wherein the dose of the monoacetyl diacylglycerol compound contained in the composition is 0.01 to 4000 mg/kg per day.
